# EUROPEAN PATENT APPLICATION

(11) **EP 4 678 752 A1**
(43) Date of publication of application: **14.01.2026**
(21) Application number: 24819602.4
(22) Date of filing: 07.06.2024
(51) Int. Cl.: C12N 15/81, C12P 23/00, C07K 14/39

(54) **YARROWIA SP. MICROORGANISM FOR PRODUCING RETINOL WITH REDUCED ABC3 PROTEIN ACTIVITY AND RETINOL PRODUCTION METHOD USING SAME**

(30) Priority: 07.06.2023 KR 20230073175
(71) Applicant: CJ Cheiljedang Corporation, Seoul 04560 (KR)
(72) Inventor: LEE, Dong Pil, Seoul 04560 (KR); KIM, Jae Eung, Seoul 04560 (KR); PARK, Hye Min, Seoul 04560 (KR); LEE, Peter, Seoul 04560 (KR); KIM, Yeonsoo, Seoul 04560 (KR); IM, Yeong Eun, Seoul 04560 (KR)
(74) Representative: Meissner Bolte Partnerschaft mbB
(86) International application number: PCT/KR2024/007785
(87) International publication number: WO 2024/253451

(57) **Abstract**

The present application relates to a *Yarrowia* sp. microorganism with retinol production ability, in which the activity of ABC3 protein is reduced compared to the endogenous activity thereof; a retinol production method using same; a method for producing *Yarrowia* sp. microorganisms with retinol production ability; a method for enhancing retinol release by microorganisms; and a composition for producing retinol.

## Description

### [Technical Field]

The present disclosure relates to a *Yarrowia* sp. microorganism having retinol-producing ability in which the activity of an ABC3 protein is reduced compared to its endogenous activity; a method for producing retinol using the same; a method for preparing a *Yarrowia* sp. microorganism having retinol-producing ability; a method for increasing retinol excretion of a microorganism; and a composition for producing retinol.

### [Background Art]

Retinol, a fat-soluble vitamin, is an essential vitamin involved in eye health for improving night blindness, strengthening immunity, skin health, and the like. Currently, it is produced and marketed mainly by leading global companies through chemical synthesis, and research is being conducted to produce retinol based on microbial fermentation. However, unlike technologies for stabilizing the retinol compound itself in a composition or product comprising retinol (US 6858217 B2), technologies for stable production of retinol have been only minimally developed.

### [Disclosure]

### [Technical Problem]

The problem to be solved by the present disclosure is to provide a *Yarrowia* sp. microorganism having retinol-producing ability in which the activity of an ABC3 protein is reduced compared to its endogenous activity; a method for producing retinol using the same; a method for preparing a *Yarrowia* sp. microorganism having retinol-producing ability; a method for increasing retinol excretion of a microorganism; and a composition for producing retinol.

### [Technical Solution]

An aspect of the present disclosure provides a *Yarrowia* sp. microorganism having retinol-producing ability in which the activity of an ABC3 protein is reduced compared to its endogenous activity.

Another aspect of the present disclosure provides a method for producing retinol, comprising culturing the microorganism of the present disclosure in a medium.

Still another aspect of the present disclosure provides a method for preparing *a Yarrowia* sp. microorganism having retinol-producing ability, comprising reducing the activity of an ABC3 protein in the *Yarrowia* sp. microorganism having retinol-producing ability compared to its endogenous activity, and a method for increasing retinol excretion.

Still another aspect of the present disclosure provides a composition for producing retinol, comprising one or more of the microorganism of the present disclosure and a culture thereof.

Still another aspect of the present disclosure provides a use of the microorganism of the present disclosure or a culture thereof for retinol production.

### [Advantageous Effects]

Retinol can be produced using the microorganism of the present disclosure.

### [Brief Description of the Drawings]

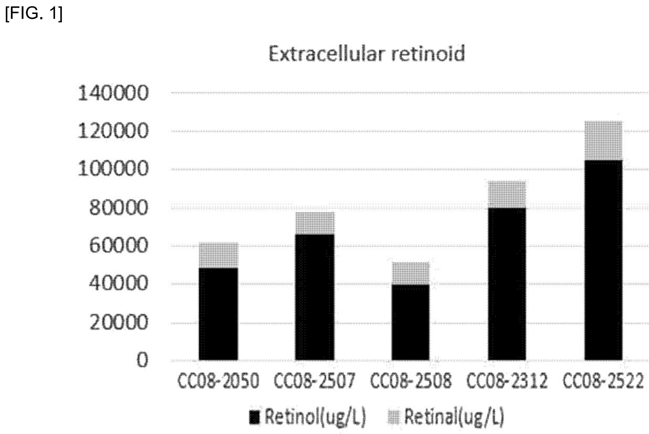

FIG. 1 is a diagram illustrating the retinol-producing ability of strains.

### [Detailed Description of Preferred Embodiments]

The present disclosure will be described in detail as follows. Meanwhile, each description and embodiment described herein can be applied to other descriptions and embodiments, respectively. That is, all combinations of various elements described herein fall within the scope of the present disclosure. Further, the scope of the present disclosure is not limited by the specific description described below. Additionally, a number of papers and patent documents have been cited throughout the present specification. The content of the cited papers and patent documents is incorporated herein by reference in their entirety to describe the level of the technical field to which the present disclosure belongs and the contents of the present disclosure more clearly.

An aspect of the present disclosure provides a *Yarrowia* sp. microorganism having retinol-producing ability in which the activity of an ABC3 (ATP-binding cassette transporter type 3) protein is reduced compared to its endogenous activity.

The reduced ABC3 protein activity may be measured by measuring the amount of ABC3 protein or polynucleotide encoding the same, or by measuring the retinol-producing ability (or yield), but the measurement is not limited thereto. For example, in the case where the retinol-producing ability of the microorganism of the present disclosure is increased compared to that of a natural wild-type *Yarrowia* sp. microorganism or a non-modified *Yarrowia* sp. microorganism (*e.g.,* a *Yarrowia* sp. microorganism expressing a wild-type polypeptide (*e.g.,* the polypeptide of SEQ ID NO: 1) having a wild-type ABC3 protein activity), the increased ABC3 protein activity may be measured by measuring the increased retinol-producing ability, but the measurement is not limited thereto.

The "ABC 3 protein (ATP-binding cassette transporter 3 protein)" of the present disclosure refers to type 3 of the ABC protein family. ABC protein is a type of ATP-binding cassette transporter protein, and can be used interchangeably with the term ABC transporter. ABC protein family comprises type 1, type 2, and type 3 proteins. Among the above, the type 3 protein, *i.e.,* ABC3 protein, is derived from a precursor having four transmembrane segments (TMS), and may have 4, 8, or 10 TMSs.

The ABC3 protein of the present disclosure may comprise YALI0B02544 protein. The YALIOB02544 protein can be used interchangeably with YALIOB02544p.

The ABC3 protein of the present disclosure may comprise any ABC3 protein that is capable of increasing retinol-producing ability and/or retinol-excreting ability. In one example, the increased retinol-producing ability may be a result of increased retinol-excreting ability.

In one embodiment, the ABC3 protein of the present disclosure may have reduced activity compared to an endogenous or wild-type ABC3 protein in a *Yarrowia* sp. microorganism, thereby increasing the retinol-producing ability and/or retinol-excreting ability of the microorganism.

In one embodiment, the ABC3 protein of the present disclosure may comprise, have, consist of, or essentially consist of an amino acid sequence having 60% or more homology or identity to the amino acid sequence of SEQ ID NO: 1.

For example, an amino acid sequence of the ABC3 protein of the present disclosure may be encoded by YALI0B02544 gene (*i.e.,* YALIOB02544g), but is not limited thereto. The amino acid sequence of the ABC3 protein may be obtained from various databases, such as NCBI's GenBank, but is not limited thereto.

In one embodiment, the ABC3 protein of the present disclosure may be derived from *Yarrowia lipolytica,* but is not limited thereto.

Additionally, while an embodiment of the ABC3 protein of the present disclosure is described as a protein comprising the amino acid sequence of SEQ ID NO: 1, the protein does not exclude addition of a non-functional sequence to an upstream or downstream region of the amino acid sequence of SEQ ID NO: 1, a naturally occurring mutation, or silent mutation thereof. It is apparent to those skilled in the art that a protein falls within the scope of the ABC3 protein the present disclosure, as long as the protein exhibits the same or corresponding activity to a protein comprising the amino acid sequence.

For example, the ABC3 protein of the present disclosure may comprise the amino acid sequence of SEQ ID NO: 1, or comprise, have, consist of, or essentially consist of an amino acid sequence having at least 60% or more, 62% or more, 63% or more, 64% or more, 62% or more, 65% or more, 70% or more, 75% or more, 76% or more, 77% or more, 78% or more, 80% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more homology or identity to the amino acid sequence of SEQ ID NO: 1. Further, it is apparent that, as long as an amino acid sequence has the above homology or identity and exhibits an efficacy corresponding to that of the protein, it falls within the scope of the present disclosure even if part of the sequence has a deletion, modification, substitution, or addition.

In one embodiment, the microorganism of the present disclosure may additionally exhibit increased SNQ2 protein activity compared to its endogenous activity.

The increased SNQ2 protein activity may be measured by measuring the amount of SNQ2 protein or polynucleotide encoding the same, or by measuring the retinol-producing ability (or yield), but the measurement is not limited thereto. For example, when, compared to the retinol-producing ability of a *Yarrowia* sp. microorganism in which the activity of the ABC3 protein of the present disclosure is reduced but the activity of SNQ2 protein is not increased or not yet increased compared to its endogenous activity, the retinol-producing ability of a microorganism in which the activity of the ABC3 protein of the present disclosure is reduced and the activity of SNQ2 protein is increased is increased, the increased retinol-producing ability may be measured to measure the increased SNQ2 protein activity, but the measurement of the increased SNQ2 protein activity is not limited thereto.

The term "SNQ2 protein (ATP-binding cassette transporter protein SNQ2)" of the present disclosure refers to a type of ATP-binding cassette transporter protein. The SNQ2 protein of the present disclosure may be a plasma membrane ABC transporter, and may be a multidrug transporter.

The SNQ2 protein of the present disclosure may include YALI0F17996 protein, and the YALIOF17996 protein may be used interchangeably with YALIOF17996p.

In one embodiment, the SNQ2 protein of the present disclosure may have an increased activity compared to an endogenous or wild-type SNQ2 protein in a *Yarrowia* sp. microorganism, thereby increasing the retinol-producing ability and/or retinol-excreting ability of the microorganism.

In one embodiment, the SNQ2 protein of the present disclosure may comprise, have, consist of, or essentially consist of an amino acid sequence having 60% or more homology or identity to the amino acid sequence of SEQ ID NO: 33.

For example, an amino acid sequence of the SNQ2 protein of the present disclosure may be encoded by YALIOF17996 gene (*i.e.,* YALI0F17996g), but is not limited thereto. The amino acid sequence of the SNQ2 protein may be obtained from various databases, such as NCBI's GenBank, but is not limited thereto.

In one embodiment, the SNQ2 protein of the present disclosure may be derived from *Yarrowia lipolytica,* but is not limited thereto.

Additionally, while an embodiment of the SNQ2 protein of the present disclosure is described as a protein comprising the sequence of SEQ ID NO: 33, the protein does not exclude addition of a non-functional sequence to an upstream or downstream region of the amino acid sequence of SEQ ID NO: 33, a naturally occurring mutation, or silent mutation thereof. It is apparent to those skilled in the art that a protein falls within the scope of the SNQ2 protein the present disclosure, as long as the protein exhibits the same or corresponding activity to a protein comprising the amino acid sequence.

For example, the SNQ2 protein of the present disclosure may comprise the amino acid sequence of SEQ ID NO: 33, or comprise, have, consist of, or essentially consist of an amino acid sequence having at least 60% or more, 62% or more, 63% or more, 64% or more, 62% or more, 65% or more, 70% or more, 75% or more, 76% or more, 77% or more, 78% or more, 80% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more homology or identity to the amino acid sequence of SEQ ID NO: 33. Further, it is apparent that, as long as an amino acid sequence has the above homology or identity and exhibits an efficacy corresponding to that of the protein, it falls within the scope of the present disclosure even if part of the sequence has a deletion, modification, substitution, or addition.

Even if the present disclosure describes 'a polypeptide (or a protein) comprising an amino acid sequence represented by a specific SEQ ID NO', 'a polypeptide (or a protein) consisting of an amino acid sequence represented by a specific SEQ ID NO', or 'a polypeptide (or a protein) having an amino acid sequence represented by a specific SEQ ID NO', it is apparent that a protein having an amino acid sequence with deletion, modification, substitution, or addition in part of the sequence may also be used in the present disclosure, as long as it has an activity identical or corresponding to that of the polypeptide or protein consisting of the amino acid sequence of the corresponding SEQ ID NO. For example, this includes cases of addition of a sequence that does not alter the function of the protein to the N-terminus and/or C-terminus of the amino acid sequence, naturally occurring mutations, silent mutations thereof, or conservative substitutions.

The term "conservative substitution" refers to the substitution of an amino acid with a different amino acid having similar structural and/or chemical properties. Such an amino acid substitution may generally occur based on similarity in polarity, charge, solubility, hydrophobicity, hydrophilicity, and/or amphipathic nature of the residues. Typically, conserved substitution may have little or no effect on the activity of a protein.

As used herein, the term "identity" or "homology'" refers to the degree of similarity between two given amino acid sequences or nucleotide sequences and may be expressed as a percentage. The terms "homology" and "identity" may often be used interchangeably in the present disclosure.

The sequence homology or identity of a conserved polynucleotide or polypeptide (including protein) is determined by standard alignment algorithms, and the default gap penalty established by the used program may be used together. Substantially, homologous or identical sequences are generally capable of hybridizing with the whole sequence or a part of the sequence, corresponding to at least about 50%, 60%, 70%, 80%, or 90% of the full length, under moderately or highly stringent conditions. It is apparent that hybridization also comprises hybridization with a polynucleotide containing a general codon or a codon considering codon degeneracy in the polynucleotide.

Whether any two polynucleotide or polypeptide (including protein) sequences have homology, similarity, or identity may be determined by, for example, a known computer algorithm such as the "FASTA" program using default parameters as in Pearson et al. (1988) [Proc. Natl. Acad. Sci. USA 85]: 2444. Alternatively, they may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ET AL.] (1988) SIAM J Applied Math 48: 1073). For example, the homology, similarity, or identity may be determined by using BLAST of the National Center for Biotechnology Information or ClustalW.

The homology, similarity, or identity between polynucleotides or polypeptides (including proteins) may be determined by, for example, comparing sequence information using a GAP computer program, such as Needleman et al., (1970), J Mol Biol. 48:443, as described in, for example, Smith and Waterman, Adv. Appl. Math (1981) 2:482. In summary, the GAP program may be defined as the value obtained by dividing the number of similarly aligned symbols (*i.e.,* nucleotides or amino acids) by the total number of the symbols in the shorter of the two sequences. The default parameters for the GAP program may include: (1) a binary comparison matrix (comprising values of 1 for identity and 0 for non-identity) and a weighted comparison matrix of Gribskov et al.. (1986) Nucl. Acids Res. 14: 6745 (or EDNAFULL (EMBOSS version of NCBI NUC4.4) substitution matrix), as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty of 10, gap extension penalty of 0.5); and (3) no penalty for end gaps.

Further, whether any two polynucleotide or polypeptide (including protein) sequences have homology, similarity, or identity may be confirmed by comparing sequences through Southern hybridization experiments under appropriate hybridization conditions, wherein the appropriate hybridization conditions are within the scope of the technical field and can be determined by methods well known to those skilled in the art (such as J. Sambrook et al., Molecular Cloning, A Laboratory Manual, 2nd Edition, Cold Spring Harbor Laboratory Press, Cold Spring Harbor, New York, 1989; F.M. Ausubel et al., Current Protocols in Molecular Biology, John Wiley & Sons, Inc., New York), but are not limited thereto.

As used herein, the term "polynucleotide", which is a polymer of nucleotides in which nucleotide monomers are connected in a long chain shape by covalent bonds, is a DNA strand having a certain length or more.

The polynucleotide sequence encoding the ABC3 protein of the present disclosure may be referred to as the YALI0B02544 gene sequence, and it may include a polynucleotide sequence encoding the amino acid sequence represented by SEQ ID NO: 1 or a degenerate sequence thereof.

The polynucleotide sequence encoding the SNQ2 protein of the present disclosure may be referred to as the YALI0F17996 gene sequence, and it may include a polynucleotide sequence encoding the amino acid sequence represented by SEQ ID NO: 33 or a degenerate sequence thereof.

In the polynucleotide encoding the SNQ2 protein of the present disclosure, various modifications may be made in the coding region as long as the amino acid sequence of the polypeptide or protein is not changed in consideration of codon degeneracy or codons preferred in organisms that are intended to express the SNQ2 polypeptide or protein.

Specifically, the polynucleotide encoding the ABC3 protein may comprise, consist of, or essentially consist of SEQ ID NO: 2 or a polynucleotide sequence having 60% or more homology or identity thereto, but is not limited thereto. For example, the polynucleotide encoding the ABC3 protein may consist of a base sequence having 60% or more, 62% or more, 63% or more, 64% or more, 62% or more, 65% or more, 70% or more, 76% or more, 77% or more, or 78% or more, 80% or more, 85% or more, 86% or more, 87% or more, 88% or more, 89% or more, 90% or more, 91% or more, 95% or more, 96% or more, 97% or more, 98% or more, or 99% or more homology or identity to the sequence of SEQ ID NO: 2, but is not limited thereto.

The polynucleotide encoding the SNQ2 protein may comprise, consist of, or essentially consist of SEQ ID NO: 34 or a polynucleotide sequence having 60% or more homology or identity thereto, but is not limited thereto. For example, the polynucleotide encoding the SNQ2 protein may consist of a base sequence having 60% or more, 62% or more, 63% or more, 64% or more, 62% or more, 65% or more, 70% or more, 76% or more, 77% or more, or 78% or more, 80% or more, 85% or more, 86% or more, 87% or more, 88% or more, 89% or more, 90% or more, 91% or more, 95% or more, 96% or more, 97% or more, 98% or more, or 99% or more homology or identity to the sequence of SEQ ID NO: 34, but is not limited thereto.

Further, the polynucleotide of the present disclosure may comprise a probe, for example, without limitation as long as it is a sequence capable of hybridizing with a complementary sequence to the entirety or part of the polynucleotide base sequence under stringent conditions.

As used herein, the term "stringent conditions" refers to conditions that enable specific hybridization between polynucleotides. These conditions are specifically described in the literature (such as J. Sambrook *et al., supra*). Examples of the stringent conditions may be conditions in which polynucleotides having high homology or identity of 40% or more, specifically 90% or more, more specifically 95% or more, 96% or more, 97% or more, or 98% or more, or even more specifically 99% or more, hybridize with each other, but polynucleotides having low homology or identity do not hybridize with each other; or washing conditions of typical Southern hybridization, i.e., conditions of washing once, or specifically two to three times, at a salt concentration and temperature corresponding to 1×SSC, 0.1% SDS at 60°C, specifically 0.1×SSC, 0.1% SDS at 60°C, or more specifically 0.1×SSC, 0.1% SDS at 68°C.

The hybridization may occur between nucleotides having complementary base sequences, but the hybridized polynucleotides may include some base mismatches depending on the stringency of the hybridization. The term "complementary" is used to describe a relationship between nucleotide bases that can hybridize with each other. For example, regarding DNA, adenine is complementary to thymine, and cytosine is complementary to guanine. Accordingly, the polynucleotide of the present disclosure may also include an isolated nucleic acid fragment complementary to the entire sequence as well as a nucleic sequence substantially similar thereto.

Specifically, polynucleotides having homology or identity may be detected using hybridization conditions including hybridization at a T m value of 55°C, as well as the above-described conditions. Further, the Tm value may be 60°C, 63°C, or 65°C, but is not limited thereto, and may be appropriately adjusted by a person skilled in the art.

The appropriate stringency for hybridizing a polynucleotide depends on the length and degree of complementarity of the polynucleotide, and the variables are well known in the relevant technical field (see J. Sambrook *et al., supra*).

For example, homologous or identical polynucleotide sequences can generally hybridize under stringent conditions along the whole sequence or at least about 50%, 60%, 70%, 80%, or 90% of the full length.

The "vector" of the present disclosure refers to a DNA construct for delivering a desired polynucleotide into a suitable host or host cell. In one example, it may comprise a base sequence of a polynucleotide encoding a desired polypeptide or protein that is operably linked to a suitable expression regulatory region (or expression control sequence) such that the desired polypeptide or protein may be expressed in a suitable host, but is not limited thereto.

The expression regulatory sequence may include a promoter capable of initiating transcription, any operator sequence for regulating the transcription, a sequence encoding a suitable mRNA ribosome-binding site, and a sequence for regulating termination of transcription and translation. The vector, after being transformed into a suitable host cell (microorganism), may replicate or function independently of the host genome, or may be integrated into the genome itself to replicate or function.

The vector of the present disclosure may be an insertion vector for inserting a polynucleotide for reducing the activity of the ABC3 protein of the present disclosure into a chromosome and/or an insertion vector for inserting a polynucleotide for increasing the activity of the SNQ2 protein of the present disclosure into a chromosome, but is not limited thereto. The insertion of the polynucleotide into the chromosome may be achieved by any method known in the art, such as homologous recombination, but is not limited thereto. The vector may further comprise a selection marker to confirm transformation into a host cell, or further, insertion into the chromosome of the host cell. The selection marker is for selecting cells transformed with vectors, or for confirming the chromosomal insertion of a desired polynucleotide, and markers that confer selectable phenotypes such as drug resistance, auxotrophy, resistance to cytotoxic agents, or expression of surface polypeptides or proteins may be used. In an environment treated with a selective agent, only cells expressing the selection marker survive or exhibit other phenotypic traits, thereby enabling selection of transformed cells. The insertion vector may not comprise an origin of replication required for replication in transformed cells.

The vector used in the present disclosure is not particularly limited, and any vector known in the art may be used. Examples of commonly used vectors include plasmids, cosmids, viruses, and bacteriophages, either in their natural state or in a recombinant state. For example, as a phage vector or cosmid vector, pWE15, M13, MBL3, MBL4, IXII, ASHII, APII, t10, t11, Charon4A, and Charon21A, *etc.* may be used; and as a plasmid vector, those based on pDZ, pBR, pUC, pBluescriptll, pGEM, pTZ, pCL, pET, *etc.* may be used. Specifically, pDZ, pDC, pDCM2, pACYC177, pACYC184, pCL, pECCG117, pUC19, pBR322, pMW118, and pCC1BAC vectors may be used.

As used herein, the term "transformation" refers to the introduction of a desired polynucleotide and/or a vector comprising the same into a host cell (microorganism), thereby altering the genetic traits of the host cell (microorganism). In the present disclosure, transformation may mean introducing a vector comprising a polynucleotide for reducing the activity of an ABC3 protein, or further comprising a polynucleotide for increasing the activity of a SNQ2 protein, into a host cell, thereby altering the genetic traits of the host cell. The transformed polynucleotide may be inserted into a chromosome of the host cell or located outside the chromosome. For example, the vector comprising a polynucleotide for reducing the activity of an ABC3 protein may be an insertion vector for insertion into the chromosome by homologous recombination to delete part or all of the YALI0B02544 gene. Further, the polynucleotide may comprise DNA and/or RNA encoding a protein. The polynucleotide may be introduced in a suitable form depending on the purpose of introduction. In addition, for example, a polynucleotide for increasing the activity of a SNQ2 protein may be introduced into a host cell in the form of an expression cassette, which is a gene construct comprising all elements necessary for self-expression. The expression cassette may comprise a promoter operably linked to the coding sequence of the SNQ protein, a transcription termination signal, a ribosome binding site, and a translation termination signal. The expression cassette may be in the form of a self-replicating expression vector. Further, the polynucleotide may be introduced into a host cell in the form *per* se and be operably linked to a sequence required for expression in the host cell, but is not limited thereto.

As used herein, the term "operably linked" refers to a constitution in which a regulatory sequence is positioned at a suitable position to control the expression of a coding sequence. Hence, "operably linked" includes a regulatory region of a functional domain with a known or desired activity, such as a promoter, terminator, signal sequence, or enhancer region, being attached or linked to a target (gene or polypeptide) to regulate its expression, secretion, or function in accordance with the known or desired activity. For example, it may mean that a polynucleotide sequence encoding a polypeptide is functionally linked to a promoter sequence that initiates and mediates transcription of the polynucleotide.

The method for transforming the vector of the present disclosure includes any method of introducing a nucleic acid into a cell and may be performed by selecting a suitable standard technique as known in the art depending on the host cell. For example, methods such as electroporation, calcium phosphate (CaPO₄) precipitation, calcium chloride (CaCl₂) precipitation, microinjection, polyethylene glycol (PEG) method, DEAE-dextran method, cationic liposome method, and lithium acetate-DMSO method may be used, but the method is not limited thereto.

As used herein, the term "microorganism (or strain)" includes both wild-type microorganisms and prokaryotic or eukaryotic microorganisms that have undergone natural or artificial genetic modification. It may be a microorganism that has undergone genetic modification for the production of a target polypeptide, protein, or product, in which a specific mechanism is weakened or reduced due to factors such as the insertion of foreign genes or the reduction or inactivation of endogenous gene activity. In the present disclosure, the terms "microorganism", "strain", "host", and "host cell" may be used interchangeably.

As used herein, the term "recombinant microorganism" refers to a microorganism that has been genetically modified to exhibit a different genotype and/or phenotype compared to a naturally occurring microorganism (*e.g.,* when the genetic modifications have an effect on a coding nucleic acid sequence of a microorganism), and may include all descendants or potential descendants of the microorganism. In the present disclosure, the terms "recombinant microorganism", "genetically modified microorganism", "recombinant host cell", "recombinant cell", and "recombinant strain" may be used interchangeably. The recombinant microorganism may, for example, express a gene that is not found in its natural (non-recombinant) form, not express a gene that is expressed in its natural form, or express a natural gene in a manner different from that in which it is expressed in its natural form.

For example, the microorganism of the present disclosure may be any one or more of: a recombinant microorganism in which ABC3 protein activity is reduced compared to its endogenous activity as a result of reduced expression of a gene encoding an ABC3 protein; and a recombinant microorganism in which SNQ2 protein activity is additionally increased compared to its endogenous activity as a result of increased expression of a gene encoding a SNQ2 protein, but is not limited thereto.

The microorganism of the present disclosure may be a microorganism having retinol-producing ability. The term "microorganism having retinol-producing ability" may be used interchangeably with "retinol-producing microorganism".

The microorganism of the present disclosure may be a microorganism into which a polynucleotide encoding lycopene cyclase/phytoene synthase (crtYB) and phytoene desaturase (crtl) proteins has been introduced so as to enable a microorganism intrinsically lacking retinol-producing ability to have retinol-producing ability, or to further increase the retinol-producing ability of a microorganism having retinol-producing ability, and thereby exhibit the activities of these proteins or have increased activities of these proteins. The lycopene cyclase/phytoene synthase or phytoene desaturase may be proteins derived from *Xanthophyllomyces dendrorhous,* but are not limited thereto as long as they are proteins that exhibit the same or similar activities. In one specific embodiment, the lycopene cyclase/phytoene synthase or phytoene desaturase may consist of or comprise the amino acid sequence of SEQ ID NO: 27 or SEQ ID NO: 29, respectively, but may also consist of or comprise an amino acid sequence having at least 60%, 65%, 70%, 75%, 80%, 85%, 86%, 87%, 88%, 89%, 90%, 91%, 92%, 93%, 94%, 95%, 96%, 97%, 98%, or 99% homology or identity to the above amino acid sequences while exhibiting an activity corresponding to the lycopene cyclase/phytoene synthase or phytoene desaturase. Further, it is apparent that a protein with deletion, modification, substitution, or addition to a part of its sequence is also included in the lycopene cyclase/phytoene synthase or phytoene desaturase, as long as the protein has the aforementioned homology or identity and exhibits an activity corresponding to said lycopene cyclase/phytoene synthase or phytoene desaturase. Further, in one specific embodiment, the polynucleotide encoding the lycopene cyclase/phytoene synthase or phytoene desaturase may consist of or comprise the sequence of SEQ ID NO: 28 or SEQ ID NO: 30, respectively. In the polynucleotide, various modifications may be made in the coding region as long as the amino acid sequence is not changed in consideration of codon degeneracy or codons preferred in the microorganism of the present disclosure. Specifically, the polynucleotide may comprise or consist of a base sequence having 60% or more, 70% or more, 80% or more, 85% or more, 90% or more, 95% or more, 96% or more, 97% or more, 98% or more, or 99% or more homology or identity to the sequence of SEQ ID NO: 28 or SEQ ID NO: 30, but is not limited thereto.

Further, the microorganism of the present disclosure may be a microorganism into which a polynucleotide encoding beta-carotene 15,15'-oxygenase (BLH) protein has been introduced so as to enable a microorganism intrinsically lacking retinol-producing ability to have retinol-producing ability, or to further increase the retinol-producing ability of a microorganism having retinol-producing ability, and thereby exhibit beta-carotene 15,15'-oxygenase activity or have increased beta-carotene 15,15'-oxygenase activity. The beta-carotene 15,15'-oxygenase may be a protein derived from uncultured marine bacterium 66A03, but is not limited thereto as long as it is a protein that exhibits the same or similar activity. In one specific embodiment, the beta-carotene 15,15'-oxygenase may consist of or comprise the amino acid sequence of SEQ ID NO: 31 but may also consist of or comprise an amino acid sequence having at least 60%, 65%, 70%, 75%, 80%, 85%, 86%, 87%, 88%, 89%, 90%, 91%, 92%, 93%, 94%, 95%, 96%, 97%, 98%, or 99% homology or identity to the amino acid sequence of SEQ ID NO: 31 while exhibiting an activity corresponding to that of the beta-carotene 15,15'-oxygenase. Further, it is apparent that a protein with deletion, modification, substitution, or addition to a part of its sequence is also included in the beta-carotene 15,15'-oxygenase, as long as the protein has the aforementioned homology or identity and exhibits an activity corresponding to the beta-carotene 15,15'-oxygenase. Additionally, in one specific embodiment, the polynucleotide encoding the beta-carotene 15,15'-oxygenase may have or comprise the sequence of SEQ ID NO: 32. In the polynucleotide, various modifications may be made in the coding region as long as the amino acid sequence is not changed in consideration of codon degeneracy or codons preferred in the microorganism of the present disclosure. Specifically, the polynucleotide may consist of or comprise a base sequence having 60% or more, 70% or more, 80% or more, 85% or more, 90% or more, 95% or more, 96% or more, 97% or more, 98% or more, or 99% or more homology or identity to the sequence of SEQ ID NO: 32, but is not limited thereto.

The CC08-2050 microorganism (identical to KCCM13294P) of the present disclosure may comprise: a lycopene cyclase/phytoene synthase comprising the amino acid sequence of SEQ ID NO: 27 and/or a polynucleotide encoding the same; a phytoene desaturase comprising the amino acid sequence of SEQ ID NO: 29 and/or a polynucleotide encoding the same; and a beta-carotene 15,15'-oxygenase comprising the amino acid sequence of SEQ ID NO: 31 and/or a polynucleotide encoding the same.

In one embodiment, the microorganism of the present disclosure may be a microorganism in which retinol-producing ability and/or retinol-excreting ability is increased as a result of reduced activity of an ABC3 protein.

The microorganism in which the activity of an ABC3 protein is reduced of the present disclosure may exhibit increased retinol-excreting ability compared to a non-modified *Yarrowia* sp. microorganism in which the activity of the ABC3 protein is not reduced, but is not limited thereto.

In one embodiment of the foregoing embodiments, the microorganism may additionally exhibit increased SNQ2 protein activity compared to its endogenous activity, but is not limited thereto.

The microorganism in which the activity of an ABC3 protein is reduced and SNQ2 protein activity is additionally increased of the present disclosure may exhibit increased retinol-excreting ability compared to a microorganism in which SNQ2 protein activity is not increased while the activity of an ABC3 protein is reduced, but is not limited thereto.

The increase in retinol-producing ability of the microorganism of the present disclosure may be a result of increasing retinol-excreting ability, but is not limited thereto.

The microorganism of the present disclosure may selectively excrete retinol, but is not limited thereto.

The microorganism of the present disclosure may selectively excrete retinol among retinal and retinol.

The microorganism of the present disclosure may be a microorganism naturally having ABC3 protein or retinol-producing ability or a parent strain having ABC3 protein or retinol-producing ability, in which the ABC3 protein of the present disclosure is reduced.

In one example, the microorganism of the present disclosure may include all microorganisms in which the activity of the ABC3 protein of the present disclosure is reduced and that are capable of producing retinol.

For example, the microorganism of the present disclosure may be a recombinant strain in which retinol-producing ability and/or retinol-excreting ability is increased as a result of reduced activity of an ABC3 protein in a natural wild-type microorganism, a microorganism having retinol-producing ability, and/or a microorganism comprising the ABC3 protein. The recombinant strain in which retinol-producing ability and/or retinol-excreting ability is increased may be a microorganism in which retinol-producing ability and/or retinol-excreting ability is increased compared to a natural wild-type microorganism or a microorganism in which the activity of the ABC3 protein of the present disclosure is not reduced, but is not limited thereto.

For example, the microorganism in which activity of the ABC3 protein of the present disclosure is not reduced and activity of a SNQ2 protein is not increased, used as a reference strain for comparing whether retinol-producing ability and/or retinol-excreting ability has increased, may be CC08-2050 (KCCM13294P, Ref. Park et al., Metabolic Engineering 2022;73:26-37), but is not limited thereto. The deposited strain name of the CJ2050 strain disclosed in the cited reference (Park et al., Metabolic Engineering 2022;73:26-37) is the CC08-2050 strain of the present disclosure, and thus, the CC08-2050 strain of the present disclosure and the CJ2050 strain are the same strain.

In one example, the recombinant strain and microorganism in which retinol-producing ability and/or retinol-excreting ability is increased may have an increased retinol-producing ability compared to the retinol-producing ability of the parent strain before modification or a non-modified microorganism by about 1% or more, about 2% or more, about 5% or more, about 10% or more, about 20% or more, or about 30% or more (the upper limit is not particularly limited and may be, for example, about 200% or less), and in another example, by about 1.01-fold or more, about 1.02-fold or more, about 1.05-fold or more, about 1.07-fold or more, about 1.1-fold or more, about 1.2-fold or more, or about 1.3-fold or more (the upper limit is not particularly limited and may be, for example, about 10-fold or less), but is not limited thereto as long as there is a positive increase compared to the producing ability of the parent strain before modification or the non-modified microorganism. The term "about" refers to a range encompassing ±0.5, ±0.4, ±0.3, ±0.2, ±0.1, *etc.,* including all numerical values in a range equal to or similar to the numerical value following the term "about", but the range is not limited thereto.

As used herein, the term "non-modified microorganism (strain)" does not exclude a microorganism (strain) comprising mutations that may occur naturally, and may refer to a wild-type microorganism (strain) or a natural microorganism (strain) *per* se, or a microorganism (strain) before a trait is altered by genetic variation due to natural or artificial factors. For example, the non-modified microorganism may refer to a microorganism (strain) in which the ABC3 protein of the present disclosure is not reduced or not yet reduced, but is not limited thereto. In the present disclosure, the term "non-modified microorganism (strain)" may be used interchangeably with "microorganism (strain) before modification", "non-mutated microorganism (strain)", "parent strain", "parent microorganism", "wild-type microorganism (strain)", or "reference microorganism (strain)".

The microorganism of the present disclosure may be a *Yarrowia* sp. microorganism, but is not limited thereto.

In one embodiment, the *Yarrowia* sp. microorganism of the present disclosure may be *Yarrowia lipolytica,* but is not limited thereto.

As used herein, the term "reduction" of the activity of a protein (polypeptide) is a concept that includes both a decrease and an inactivation of the activity of the protein (polypeptide) in a host cell (microorganism) compared to its endogenous activity.

That is, the reduction of the activity of a protein (polypeptide) may include both a state in which the protein (polypeptide) in a host cell (microorganism) is not completely inactivated and thus exhibits reduced protein (polypeptide) activity compared to its endogenous or pre-modification activity; or a state in which the activity of the protein (polypeptide) is completely inactivated.

For example, the reduction may also include cases where: the protein (polypeptide) activity is low or absent compared to the protein (polypeptide) originally possessed by the host cell (microorganism) before transformation or a non-modified host cell (microorganism) due to mutation of the polynucleotide encoding the protein (polypeptide), *etc.;* the overall intracellular expression level of the protein (polypeptide) is decreased compared to the host cell (microorganism) before transformation or a non-modified host cell (microorganism) due to the inhibition of expression of the polynucleotide or the protein (polypeptide), *etc.;* the polynucleotide or the protein (polypeptide) is not expressed at all; and the protein (polypeptide) activity is low or absent even though the protein (polypeptide) expression occurs normally.

The host cell (microorganism) may be a prokaryotic or eukaryotic microorganism.

A reduction in the activity of a protein (polypeptide) compared to its endogenous activity means that the activity and/or concentration (expression level) of the protein (polypeptide) in a host cell (microorganism) is decreased compared to the activity and/or concentration (expression level) of the protein (polypeptide) originally possessed by the host cell (microorganism) before modification or a non-modified host cell (microorganism).

Whether activity of a protein (polypeptide) has reduced may be confirmed from the degree of activity of the protein (polypeptide), its expression level, or the amount of product resulting from the activity of the protein (polypeptide).

The "endogenous activity" refers to the activity of a specific protein (polypeptide) originally possessed by a host cell (microorganism) before transformation or a non-modified host cell (microorganism), when the trait is altered by genetic variation due to natural or artificial factors. This term may be used interchangeably with "activity before modification".

The microorganism of the present disclosure may exhibit enhanced retinoid-producing ability and/or retinoid-excreting ability as a result of weakening of the activity of an ABC3 protein.

The reduction in the activity of a protein (polypeptide) can be achieved by various methods well known in the art, and is not limited as long as the activity of the desired protein (polypeptide) can be reduced compared to that of the host cell (microorganism) before modification. Specifically, it may be using genetic engineering and/or protein engineering well known to a person of ordinary skill in the art, which are routine molecular biology techniques, but is not limited thereto (*e.g.,* Nakashima N et al., Bacterial cellular engineering by genome editing and gene silencing. Int J Mol Sci. 2014;15(2):2773-2793; Sambrook et al. Molecular Cloning 2012; *etc*.).

Specifically, the reduction in the activity of a protein (polypeptide) of the present disclosure may result from:
1) deletion of part or all of a gene encoding the protein (polypeptide);
2) modification of a gene expression regulatory region (*e.g.,* introduction of a mutation in the expression regulatory region, replacement with a sequence exhibiting expression-inhibiting activity, or insertion of a sequence exhibiting expression-inhibiting activity) on a chromosome encoding the protein (polypeptide);
3) modification of the amino acid sequence of the protein (polypeptide) (*e.g.,* deletion/substitution/addition of one or more amino acids in the amino acid sequence) such that the protein (polypeptide) activity is reduced;
4) modification of a polynucleotide sequence encoding the protein (polypeptide) (*e.g.,* modification of a polynucleotide sequence of a gene encoding a protein (polypeptide), such that the gene encodes the protein (polypeptide) modified to have reduced protein (polypeptide) activity) such that the activity of the protein (polypeptide) is reduced;
5) modification of a base sequence encoding the initiation codon or 5'-UTR region of the gene encoding the protein (polypeptide);
6) introduction of an antisense oligonucleotide (*e.g.,* antisense RNA) that binds complementarily to the gene transcript encoding the protein (polypeptide);
7) addition of a sequence complementary to the Shine-Dalgarno (SD) sequence upstream of the SD sequence of the gene encoding the protein (polypeptide) to form a secondary structure that prevents ribosomal attachment;
8) reverse transcription engineering (RTE), which adds a promoter to be reversely transcribed on the 3' terminus of the open reading frame (ORF) of the polynucleotide sequence encoding the protein (polypeptide);
9) regulation of the cellular localization of the protein (polypeptide); or
10) a combination of two or more selected from 1) to 9) above, but is not particularly limited thereto.

For example,

The 1) deletion of part or all of a gene encoding the protein (polypeptide) may be achieved by a method involving homologous recombination using a chromosome insertion vector within the microorganism, and/or by using exposure to electromagnetic waves such as ultraviolet or radiation or treatment with chemical agents, but is not limited thereto.

The 2) modification of a gene expression regulatory region on a chromosome encoding the protein (polypeptide) may be achieved, for example, by introducing a modification in the gene expression regulatory region through deletion, insertion, substitution, or a combination thereof to further reduce expression-inducing activity of the expression regulatory region, or by replacing the sequence with a sequence having further expression-inhibiting activity. The expression regulatory region may comprise, but is not particularly limited to, a promoter, an operator sequence, a sequence encoding a ribosome binding site, a sequence controlling the termination of transcription and translation, *etc.*

The 3) and 4) modification of the amino acid sequence or the polynucleotide sequence of the protein (polypeptide) may involve introducing a mutation in the sequence by deletion, insertion, substitution, or a combination thereof in the amino acid sequence of the protein (polypeptide) or the polynucleotide sequence encoding the protein (polypeptide) to reduce the activity of the protein (polypeptide), or replacing it with an amino acid sequence or polynucleotide sequence modified to reduce the activity, but are not limited thereto. The sequence modification may be achieved, for example, by inserting a polynucleotide with a modified sequence into a chromosome through homologous recombination, but is not limited thereto. In one specific embodiment, by introducing a mutation in the polynucleotide sequence encoding the protein (polypeptide) to form a termination codon, the protein (polypeptide) may be inactivated, but the modification is not limited thereto.

The 5) modification of a base sequence encoding a start codon or 5'-UTR of a gene encoding the protein (polypeptide) may involve, for example, substituting with another start codon with a lower protein (polypeptide) expression rate as compared to an endogenous start codon, or a modification to encode a ribosome binding site (RBS) sequence with a lower protein (polypeptide) expression rate as compared to an endogenous RBS sequence, but is not limited thereto.

The 6) introduction of an antisense oligonucleotide (*e.g.*, antisense RNA) that binds complementarily to the gene transcript encoding the protein (polypeptide) may be achieved in reference to the literature [Weintraub, H. et al., Antisense-RNA as a molecular tool for genetic analysis, Reviews - Trends in Genetics, Vol. 1(1) 1986], but is not limited thereto.

The 7) addition of a sequence complementary to the Shine-Dalgarno (SD) sequence upstream of the SD sequence of the gene encoding the protein (polypeptide) to form a secondary structure that prevents ribosomal attachment may be preventing mRNA translation or reducing the speed thereof, but is not limited thereto.

Further, the 8) reverse transcription engineering (RTE), which adds a promoter to be reversely transcribed on the 3' terminus of the open reading frame (ORF) of the polynucleotide sequence encoding the protein (polypeptide) may be achieved by generating an antisense nucleotide complementary to the gene transcript encoding the polypeptide to inhibit the translation of the protein (polypeptide) and reduce the activity.

The method of 9) regulation of the cellular localization of the protein (polypeptide) may be targeting the protein (polypeptide) to a particular organelle or a particular space within the cell. For example, it may be targeting the protein (polypeptide) to the periplasm or cytoplasm through addition or removal of a leader sequence that functions in protein targeting, but is not limited thereto.

Such reduction in the protein (polypeptide) activity may result in a reduction in the activity or concentration of the corresponding protein (polypeptide) as compared to the activity or concentration of the protein (polypeptide) expressed in a wild-type host cell (microorganism) or a host cell (microorganism) before modification, but is not limited thereto.

The modification of a part or the entirety of a polynucleotide in the host cell (microorganism) of the present disclosure may be induced by: (a) a method using homologous recombination using a vector for chromosomal insertion or genome editing using an engineered nuclease (*e.g*., CRISPR-Cas9), and/or (b) treatment with light, such as ultraviolet light and radiation, and/or chemicals, but is not limited thereto.

In one embodiment, the microorganism of the present disclosure may have deletion of part or all of the gene sequence encoding an ABC3 protein, but is not limited thereto.

As used herein, an "increase" in protein (polypeptide) activity means that the activity of a protein (polypeptide) is increased compared to its endogenous activity in a host cell (microorganism). The increase may be used interchangeably with terms such as "activation", "up-regulation", "overexpression", or "enhancement".

The host cell (microorganism) may be a prokaryotic or eukaryotic microorganism.

The increase in protein (polypeptide) activity may include a host cell (microorganism) both exhibiting a protein (polypeptide) activity that it did not intrinsically possess, or exhibiting an improved protein (polypeptide) activity compared to its endogenous activity or activity before modification.

For example, the "exhibiting a protein (polypeptide) activity that it did not intrinsically possess" or "exhibiting an improved protein (polypeptide) activity" may be due to "introduction of a protein (polypeptide)", but is not limited thereto. The introduction of a protein (polypeptide) may be due to introduction of a gene encoding the protein (polypeptide) into a host cell (microorganism). For example, a polynucleotide encoding a specific protein (polypeptide) may be introduced into a chromosome of a host cell (microorganism), or a vector comprising the polynucleotide encoding a specific protein (polypeptide) may be introduced into the host cell (microorganism), thereby exhibiting or enhancing the activity thereof.

An increase in activity of a protein (polypeptide) compared to its endogenous activity means that the activity and/or concentration (expression level) of the protein (polypeptide) in a host cell (microorganism) is enhanced compared to the activity and/or concentration (expression level) of the protein (polypeptide) originally possessed by the host cell (microorganism) before modification or a non-modified host cell (microorganism).

The increase in activity of a protein (polypeptide) can be achieved by introducing a foreign protein (polypeptide) or by increasing the activity of an endogenous protein (polypeptide). Whether activity of a protein (polypeptide) has increased may be confirmed from the increase in the degree of activity of the protein (polypeptide), its expression level, or the amount of product resulting from the activity of the protein (polypeptide).

The increase in the activity of a protein (polypeptide) can be achieved by various methods well known in the art, and is not limited as long as the activity of the desired protein (polypeptide) can be increased compared to that of the host cell (microorganism) before modification. Specifically, genetic engineering and/or protein engineering well known to those skilled in the art as routine methods in molecular biology may be used, but the method is not limited thereto (e.g., Sitnicka et al. Functional Analysis of Genes. Advances in Cell Biology. 2010, Vol. 2. 1-16, Sambrook et al. Molecular Cloning 2012, *etc*.).

Specifically, the increase in the activity of a protein (polypeptide) of the present disclosure may result from:
1) increase in the intracellular copy number of a polynucleotide encoding the protein (polypeptide);
2) modification of a gene expression regulatory region on a chromosome encoding the protein (polypeptide) (*e.g*., introduction of a mutation in the expression regulatory region, replacement with a sequence exhibiting strong expression-inducing activity, or insertion of a sequence exhibiting strong expression-inducing activity);
3) modification of a base sequence encoding a start codon or the 5'-UTR of a gene transcript encoding the protein (polypeptide);
4) modification of an amino acid sequence of the protein (polypeptide) to increase the activity of the protein (polypeptide);
5) modification of a polynucleotide sequence encoding the protein (polypeptide) to increase the activity of the protein (polypeptide) (*e.g.,* modification of a polynucleotide sequence of a gene encoding the protein (polypeptide), such that the gene encodes a protein (polypeptide) modified to have increased activity);
6) introduction of a foreign protein (polypeptide) exhibiting the activity of the protein (polypeptide), or a foreign polynucleotide encoding the same;
7) codon optimization of a polynucleotide encoding the protein (polypeptide);
8) selection and modification or chemical modification of exposed regions of the protein (polypeptide) through an analysis of tertiary structure thereof;
9) regulating the cellular localization of the protein (polypeptide); or
10) a combination of two or more selected from 1) to 9) above, but is not particularly limited thereto.

For example,

The 1) increase in the intracellular copy number of a polynucleotide encoding the protein (polypeptide) may involve introducing a polynucleotide encoding the protein (polypeptide) operably linked to an appropriate regulatory sequence in the form of a vector comprising the same into a host cell (microorganism). Alternatively, the method may be achieved by introducing one copy, or two or more copies, of a polynucleotide encoding the protein (polypeptide) operably linked to a suitable regulatory sequence into the chromosome of a host cell (microorganism). The introduction into the chromosome may be performed by introducing a vector, which is able to insert the polynucleotide into the chromosome of a host cell (microorganism), into the host cell (microorganism), but is not limited thereto. The vector is as described above. With respect to the polynucleotide sequence encoding the protein, the regulatory sequence may be a native sequence (of the same origin) or a foreign sequence (derived from a different gene), a variant thereof, or another artificial sequence, and may induce expression of the polynucleotide in a host cell (microorganism).

The 2) replacement of a gene expression regulatory region (or expression control sequence) on a chromosome encoding the protein (polypeptide) with a sequence exhibiting strong expression-inducing activity may involve, for example, introducing a mutation in a sequence by deletion, insertion, substitution, or a combination thereof such that the expression-inducing activity of the expression regulatory region is further enhanced, or a replacement with a sequence exhibiting a stronger expression-inducing activity. The expression regulatory region may include, but is not particularly limited to, a promoter, an operator sequence, a sequence encoding a ribosome binding site, and a sequence regulating the termination of transcription and translation. In one example, it may involve replacing the original promoter with a promoter exhibiting a strong expression-inducing activity, but is not limited thereto.

Examples of known promoters exhibiting strong expression-inducing activity comprise cj1 to cj7 promoters (US 7662943 B2), a lac promoter, a trp promoter, a trc promoter, a tac promoter, a lambda phage PR promoter, a PL promoter, a tet promoter, a gapA promoter, a SPL7 promoter, a SPL13(sm3) promoter (US 10584338 B2), an O2 promoter (US 10273491 B2), a tkt promoter, a yccA promoter, a TEF promoter, etc., but are not limited thereto.

The 3) modification of a base sequence encoding a start codon or 5'-UTR of a gene encoding the protein (polypeptide) may involve, for example, substituting with another start codon with a higher protein (polypeptide) expression rate as compared to an endogenous start codon, or a modification to encode for a ribosome binding site (RBS) sequence with a higher protein (polypeptide) expression rate as compared to an endogenous RBS sequence, but is not limited thereto.

The 4) and 5) modification of the amino acid sequence or the polynucleotide sequence of the protein (polypeptide) may involve introducing a mutation in the sequence by deletion, insertion, substitution, or a combination thereof in the amino acid sequence of the protein (polypeptide) or the polynucleotide sequence encoding the protein (polypeptide), or replacing with an amino acid sequence or polynucleotide sequence modified to exhibit increased activity, but is not limited thereto. The sequence modification may be achieved, for example, by inserting a polynucleotide with a modified sequence into a chromosome through homologous recombination, but is not limited thereto.

The method of 6) introducing a foreign polynucleotide exhibiting the activity of the protein (polypeptide) may be achieved by introducing into a host cell (microorganism) a foreign polynucleotide encoding a protein (polypeptide) that exhibits the same/similar activity to that of the protein (polypeptide). The foreign polynucleotide is not limited by its origin or sequence, as long as it exhibits the same/similar activity to that of the protein (polypeptide). The introduction may be performed by a transformation method known in the art appropriately selected by a person of ordinary skill in the art, and the expression of the introduced polynucleotide in the host cell enables the production of the protein (polypeptide), thereby increasing its activity.

The 7) codon optimization of a polynucleotide encoding the protein (polypeptide) may be performed such that the transcription or translation of an endogenous polynucleotide is increased in a host cell (microorganism), or such that the transcription or translation of a foreign polynucleotide is optimized in a host cell (microorganism).

The 8) selection and modification or chemical modification of exposed regions of the protein (polypeptide) through an analysis of tertiary structure thereof may involve, for example, determining a template protein candidate based on the degree of similarity of the sequence by comparing the sequence information of a protein (polypeptide) to be analyzed with a database storing the sequence information of known proteins, confirming the structure based thereon, selecting an exposed site to be modified or chemically modified, and modifying or chemically modifying the same.

The 9) regulation of the cellular localization of the protein (polypeptide) may involve targeting the protein (polypeptide) to a specific cellular organelle or a specific intracellular space. For example, it may involve targeting a protein (polypeptide) to the periplasm or cytoplasm by adding or removing a leader sequence that functions in the targeting of the protein (polypeptide), but is not limited thereto.

Such increase in the protein (polypeptide) activity may result in an increase in the activity or concentration of the corresponding protein (polypeptide) as compared to the activity or concentration of the protein (polypeptide) expressed in a wild-type host cell (microorganism) or a host cell (microorganism) before modification, but is not limited thereto.

In one embodiment, the microorganism of the present disclosure may exhibit strong SNQ2 protein expression-inducing activity by reducing the activity of an ABC3 protein and additionally exchanging a promoter sequence of a gene encoding a SNQ2 protein with a TEF promoter.

In the present disclosure, "retinol" refers to the compound known as vitamin A. In one embodiment, retinol can be converted into other retinoid compounds (e.g., retinal, retinoic acid, and retinyl ester) by methods known in the art.

In the present disclosure, "retinoid" refers to a group of compounds chemically belonging to the vitamin A family or chemically related thereto.

In one embodiment, the retinoid may be any one selected from the group consisting of retinol, retinal, retinoic acid, and retinyl ester, but is not limited thereto.

Additionally, the microorganism of the present disclosure may have or have increased retinoid-producing ability by further comprising a substance (*e.g.,* an enzyme) that converts retinol into other retinoids (*e.g.,* retinoic acid and retinyl ester, *etc*.).

Another aspect of the present disclosure provides a method for producing retinol, comprising culturing a *Yarrowia* sp. microorganism having retinol-producing ability in which the activity of an ABC3 protein is reduced compared to its endogenous activity, in a medium.

The microorganism is as described in another aspect.

In one embodiment, the microorganism may additionally exhibit increased SNQ2 protein activity compared to its endogenous activity, but is not limited thereto.

As used herein, the term "cultivation" means growing the microorganism of the present disclosure under appropriately controlled environmental conditions. The cultivation process of the present disclosure may be performed according to suitable medium and cultivation conditions known in the art. Such a cultivation process may be easily adjusted and used by those skilled in the art according to the selected strain. Specifically, the cultivation may be a batch type, continuous type, and/or fed-batch type, but is not limited thereto.

The microorganism of the present disclosure may be cultured in a common medium containing appropriate carbon sources, nitrogen sources, phosphorus sources, inorganic compounds, amino acids and/or vitamins, *etc.,* while controlling the temperature, pH, *etc.* under aerobic conditions.

In the present disclosure, the carbon sources may comprise carbohydrates such as glucose, saccharose, lactose, fructose, sucrose, maltose, *etc.;* sugar alcohols such as mannitol, sorbitol, *etc.;* organic acids such as pyruvic acid, lactic acid, citric acid, *etc*.; and amino acids such as glutamic acid, methionine, lysine, *etc.* Further, natural organic nutrient sources such as starch hydrolysate, molasses, blackstrap molasses, rice bran, cassava, sugarcane residue, and corn steep liquor may be used. Specifically, carbohydrates such as glucose and sterilized pretreated molasses (*i.e.*, molasses converted to reducing sugar) may be used, and appropriate amounts of other carbon sources may be used in various manners without limitation. These carbon sources may be used alone or in combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc.;* and organic nitrogen sources, such as peptone, NZ-amine, meat extracts, yeast extracts, malt extracts, corn steep liquor, casein hydrolysates, fish or decomposition products thereof, defatted soybean cake or degradation products thereof, *etc.* may be used. These nitrogen sources may be used alone or in a combination of two or more thereof, but are not limited thereto.

The phosphorus sources may comprise monopotassium phosphate, dipotassium phosphate, or sodium-containing salts corresponding thereto. As for inorganic compounds, sodium chloride, calcium chloride, iron chloride, magnesium sulfate, iron sulfate, manganese sulfate, calcium carbonate, *etc.* may be used, and in addition, amino acids, vitamins, and/or suitable precursors may be included. These constituents or precursors may be added to the medium in a batch or continuous manner, but are not limited thereto. However, the medium is not limited thereto.

During the cultivation of the microorganism of the present disclosure, the pH of the medium may be adjusted by adding compounds such as ammonium hydroxide, potassium hydroxide, ammonia, phosphoric acid, sulfuric acid, *etc.* to the medium in a suitable manner. Further, during the cultivation, foaming may be suppressed by using an antifoaming agent such as fatty acid polyglycol ester. Additionally, oxygen or oxygen-containing gas may be injected into the medium in order to maintain the aerobic state of the medium, or gas may not be injected or nitrogen, hydrogen, or carbon dioxide gas may be injected in order to maintain the anaerobic and microaerobic states, but are not limited thereto.

Further, the culture medium may contain metal salts such as magnesium sulfate or iron sulfate necessary for growth. Lastly, in addition to the aforementioned substances, essential growth substances such as amino acids and vitamins may be used. Additionally, suitable precursors may be used in the culture medium. The aforementioned raw materials may be added to the culture in a batch or continuous manner by a suitable method during the cultivation process, but are not limited thereto.

During the cultivation of the microorganism of the present disclosure, the pH of the culture may be adjusted by adding compounds such as ammonium hydroxide, potassium hydroxide, ammonia, phosphoric acid, sulfuric acid, *etc.* to the culture in a suitable manner. Further, during the cultivation, foaming may be suppressed by using an antifoaming agent such as fatty acid polyglycol ester. Additionally, oxygen or oxygen-containing gas may be injected into the culture in order to maintain the aerobic state of the culture, or gas may not be injected or nitrogen, hydrogen, or carbon dioxide gas may be injected in order to maintain the anaerobic and microaerobic states, but are not limited thereto.

In the cultivation of the present disclosure, the cultivation temperature may be maintained at 20°C to 35°C, specifically 25°C to 35°C. The cultivation period may be continued until the amount of the useful substance is obtained, and may be about 10 hours to 160 hours, about 20 hours to 130 hours, about 24 hours to 120 hours, about 36 hours to 120 hours, about 48 hours to 120 hours, about 48 hours or more, or about 48 hours, about 72 hours, or about 120 hours, but is not limited thereto.

The method for producing retinol of the present disclosure may further comprise recovery of retinol from the microorganism or the medium.

The desired retinol may be recovered from the medium by using an appropriate method known in the art according to the cultivation method of the microorganism of the present disclosure, for example, a batch, continuous, or fed-batch type cultivation method. For example, centrifugation, filtration, treatment with a crystallized protein precipitating agent (salting-out), extraction, ultrafiltration, dialysis, various types of chromatography such as molecular sieve chromatography (gel filtration), adsorption chromatography, ion exchange chromatography, affinity chromatography, *etc.,* HPLC, and a combination of these methods may be used, but is not limited thereto.

The method for producing retinol may further comprise a purification process.

The purification process may be performed by using an appropriate method known in the art.

In one embodiment, the method for producing retinol of the present disclosure uses a microorganism having reduced ABC3 protein activity and having retinol-excreting ability, and thus, the present disclosure can produce retinol without employing cell disruption of microorganisms, which is widely employed for retinol extraction, or without using dodecane as a solvent, but is not limited thereto.

The method for producing retinol of the present disclosure may further comprise conversion of retinol expressed by the microorganism of the present disclosure into a retinoid other than retinol; accordingly, the present disclosure may provide a method for producing a retinoid. With regard to the method for producing retinoid of the present disclosure, the conversion may be further comprised after the cultivation of the microorganism or the recovery of retinol. The conversion may be performed by using an appropriate method known in the art. For example, the conversion may be performed using retinol acyltransferase, but is not limited thereto.

In one embodiment, the retinoid other than retinol may be any one selected from the group consisting of retinal, retinoic acid, and retinyl ester, but is not limited thereto as long as it is included in retinoids.

Still another aspect of the present disclosure provides a method for preparing *a Yarrowia* sp. microorganism having retinol-producing ability, comprising reducing the activity of an ABC3 protein in a *Yarrowia* sp. microorganism having retinol-producing ability.

Yet another aspect of the present disclosure provides a method for increasing retinol excretion, comprising reducing the activity of an ABC3 protein in a *Yarrowia* sp. microorganism having retinol-producing ability.

In one embodiment, the method for preparing a *Yarrowia* sp. microorganism and the method for increasing retinol excretion may further comprise increasing SNQ2 protein activity in the microorganism, but are not limited thereto.

The step of reducing the activity of an ABC3 protein may involve modifying a *Yarrowia* sp. microorganism such that the activity of the ABC3 protein is reduced compared to its endogenous activity, and the step of increasing the activity of a SNQ2 protein may involve modifying a *Yarrowia* sp. microorganism such that the activity of the SNQ2 protein is increased compared to its endogenous activity, as described in other aspects.

Yet another aspect of the present disclosure provides a composition for producing retinol comprising one or more of a *Yarrowia* sp. microorganism having retinol-producing ability in which the activity of an ABC3 protein is reduced compared to its endogenous activity, and a culture thereof.

In one embodiment, the microorganism may additionally exhibit increased SNQ2 protein activity compared to its endogenous activity, but is not limited thereto.

The composition of the present disclosure may further comprise any appropriate excipient that is commonly used in compositions for retinol production. Such excipients may be, for example, a preservative, a wetting agent, a dispersing agent, a suspending agent, a buffering agent, a stabilizing agent, or an isotonic agent, *etc.,* but are not limited thereto.

Additionally, the present disclosure may provide a composition for producing retinoid by further comprising a substance for converting retinol into retinoid other than retinol (e.g., enzymes such as retinol acyltransferase) in the composition for producing retinol.

The ABC3 protein, the *Yarrowia* sp. microorganism in which the activity of an ABC3 protein is reduced, the SNQ2 protein, the *Yarrowia* sp. microorganism in which the activity of a SNQ2 protein is increased, the retinol, and the retinoid, *etc.,* are as described in other aspects.

Yet another aspect of the present disclosure provides a use of a *Yarrowia* sp. microorganism in which the activity of an ABC3 protein is reduced compared to its endogenous activity or a culture thereof for retinol production.

Yet another aspect of the present disclosure provides a use of a *Yarrowia* sp. microorganism in which the activity of an ABC3 protein is reduced compared to its endogenous activity.

In one embodiment, with respect to the use for retinol production and use for retinoid production, the microorganism may additionally exhibit increased SNQ2 protein activity compared to its endogenous activity, but is not limited thereto.

The ABC3 protein, the *Yarrowia* sp. microorganism in which the activity of an ABC3 protein is reduced, the SNQ2 protein, the *Yarrowia* sp. microorganism in which the activity of a SNQ2 protein is increased, the retinol, and the retinoid, *etc.,* are as described in other aspects.

### [Mode for Carrying Out the Invention]

The present disclosure will be described in detail by way of Examples. However, these Examples are given for illustrative purposes only, and the scope of the invention is not intended to be limited by these Examples. Meanwhile, technical matters not described in the present specification may be sufficiently understood and easily implemented by those skilled in the technical field of the present disclosure or similar technical fields.

### Example 1. Construction of a Yarrowia spp. producing retinol with reduced ABC3 activity

In KCCM13294P (CC08-2050, Park et al., Metab Eng.2022, Jun 6;73:26-37), *a Yarrowia* strain producing retinol, the protein activity of ABC3 (YALI0B02544) was reduced.

To this end, the ORF sequence (SEQ ID NO: 2) of the ABC gene (YALIOB02544g) was obtained based on the nucleotide sequence registered in KEGG (Kyoto Encyclopedia of Genes and Genomes). Additionally, a deletion cassette of the ABC3 gene (YALI0B02544g) was constructed using the URA3 gene (SEQ ID NO: 4) of *Y. lipolytica* as a selection marker. At this time, the genomic DNA of CC08-2050 was used as a template and PCR was performed using the primers of SEQ ID NO: 5 and SEQ ID NO: 6, SEQ ID NO: 7 and SEQ ID NO: 8, SEQ ID NO: 9 and SEQ ID NO: 10, and SEQ ID NO: 11 and SEQ ID NO: 12 on the left homologous region, URA3, repeat region, and right homologous region fragments, respectively. The PCR conditions consisted of denaturation at 95°C for 1 minute, annealing at 55°C for 1 minute, and polymerization at 72°C for 2 minutes, repeated for 35 cycles. The obtained DNA fragments were assembled into a single cassette through overlap extension PCR. The cassette thus constructed was introduced into the CC08-2050 strain using the heat shock method (D.-C. Chen et al., Appl Microbiol Biotechnol, 1997), and colonies formed on a solid medium (YLMM1) lacking uracil were obtained. The colonies in which cassette insertion into the genome was confirmed using primers of SEQ ID NO: 13 and SEQ ID NO: 14 were cultured on 5-FOA solid medium at 30°C for 3 days, and by obtaining colonies grown on the 5-FOA solid medium, the URA3 marker was recovered. Thus-obtained final strain with reduced ABC3 protein activity was named CC08-2507.

**[Table 1]**

| SEQ ID NO | Sequence (5'- 3') | PCR Product |
|---|---|---|
| 5 | GTCCACTTCTACAGCAACATTATG | Homology left arm |
| 6 | GACGAGTCAGACAGGAGGCAGGTACGATAGTA AAGTCTGTTGATAC | |
| 7 | GTATCAACAGACTTTACTATCGTACCTGCCTCCT GTCTGACTCGTC | URA3 |
| 8 | AATGGTTGGATGAGGGTGGATGGTGGTATTGTG ACTGGGG | |
| 9 | CCCCAGTCACAATACCACCATCCACCCTCATCC AACCATT | Repeat region |
| 10 | GCCACAGTTTTGACGTAATAAGCGGTACGATAG TAAAGTCTGT | |
| 11 | ACAGACTTTACTATCGTACCGCTTATTACGTCAA AACTGTGGC | Homology right arm |
| 12 | GAGATGAACTGTCCAAATCGAG | |
| 13 | GTATGTGCCTGGATCGTACC | Check forward |
| 14 | CAACAACTCGGGATCCACTC | Check reverse |

The aforementioned YLMM1 and 5-FOA media used had the following compositions:
*<Yarrowia lipolytica* Minimal Medium 1 (YLMM1)>

Glucose 20 g/L, Yeast nitrogen base without amino acids 6.7 g/L, Yeast Synthetic Drop-out Medium Supplements without uracil 2 g/L, Agar 15 g/L
<5-Fluoroorotic Acid (5-FOA) Medium>

Glucose 20 g/L, Yeast nitrogen base without amino acids 6.7 g/L, Yeast Synthetic Drop-out Medium Supplements without uracil 2 g/L, Uracil 50 µg/mL, 5-Fluoroorotic acid (5-FOA) 1 g/L, Agar 15 g/L

### Example 2. Construction of a Yarrowia spp. producing retinol with increased ABC3 activity

The ABC3 (YALIOB02544) protein activity of KCCM13294P, which is a *Yarrowia* strain producing retinol, was increased. To replace the promoter of the ABC3 gene (YALI0B02544g), the ORF sequence (SEQ ID NO: 2) of the ABC gene (YALIOB02544g) was obtained based on the nucleotide sequence registered in KEGG (Kyoto Encyclopedia of Genes and Genomes). Additionally, a promoter replacement cassette was constructed using the URA3 gene (SEQ ID NO: 4) of *Y. lipolytica* as a selection marker. The sequence of the TEF promoter corresponds to SEQ ID NO: 3. At this time, the genomic DNA of CC08-2050 was used as a template and PCR was performed using the primers of SEQ ID NO: 15 and SEQ ID NO: 16, SEQ ID NO: 17 and SEQ ID NO: 18, SEQ ID NO: 19 and SEQ ID NO: 20, SEQ ID NO: 21 and SEQ ID NO: 22, and SEQ ID NO: 23 and SEQ ID NO: 24 on the left homologous region, TEF promoter, URA3, repeat region, and right homologous region fragments, respectively. The PCR conditions consisted of denaturation at 95°C for 1 minute, annealing at 55°C for 1 minute, and polymerization at 72°C for 2 minutes, repeated for 35 cycles. The obtained DNA fragments were assembled into a single cassette through overlap extension PCR. The cassette thus constructed was introduced into the CC08-2050 strain using the heat shock method (D.-C. Chen et al., Appl Microbiol Biotechnol, 1997), and colonies formed on a solid medium (YLMM1) lacking uracil were obtained. The insertion of the cassette into the genome was confirmed using primers of SEQ ID NO: 25 and SEQ ID NO: 26. The obtained colonies were cultured on 5-FOA solid medium at 30°C for 3 days, and by obtaining colonies grown on the 5-FOA solid medium, the URA3 marker was recovered. Thus-obtained final strain with increased ABC3 protein activity was named CC08-2508.

**[Table 2]**

| SEQ ID NO | Sequence (5'- 3') | PCR Product |
|---|---|---|
| 15 | GTCCACTTCTACAGCAACATTATG | Homology left arm |
| 16 | CGCCGCCAACCCGGTCTCTGGTACGATAGTAAAGT CTGTTGATAC | |
| 17 | GTATCAACAGACTTTACTATCGTACCAGAGACCGG GTTGGCGGCG | TEF promoter |
| 18 | GACGAGTCAGACAGGAGGCATTTGAATGATTCTTA TACTC | |
| 19 | GAGTATAAGAATCATTCAAATGCCTCCTGTCTGACT CGTC | URA3 |
| 20 | GCGCCGCCAACCCGGTCTCTTGGTGGTATTGTGAC TGGGG | |
| 21 | CCCCAGTCACAATACCACCAAGAGACCGGGTTGGC GGCGC | Repeat region |
| 22 | GTGATCGGTACAGGATCTGTCATTTTGAATGATTCT TATACTCAGAAGG | |
| 23 | CCTTCTGAGTATAAGAATCATTCAAAATGACAGATC CTGTACCGATCAC | Homology right arm |
| 24 | CACGTCTCGAGTATGCTTTGC | |
| 25 | GTATGTGCCTGGATCGTACC | Check forward |
| 26 | CAATACTCTCGGCGATAGAGAC | Check reverse |

The aforementioned YLMM1 and 5-FOA media used had the same compositions as described in Example 1.

### Example 3. Construction of a Yarrowia spp. producing retinol with increased SNQ2 activity

The SNQ2 (YALI0F17996) protein activity of KCCM13294P (CC08-2050), a *Yarrowia* strain producing retinol, and CC08-2507 constructed in Example 1 was increased. To this end, the ORF sequence (SEQ ID NO: 34) of the ABC gene (YALI0F17996g) was obtained based on the nucleotide sequence registered in KEGG (Kyoto Encyclopedia of Genes and Genomes). Additionally, a promoter replacement cassette of the SNQ2 gene (YALI0F17996g) was constructed using the URA3 gene (SEQ ID NO: 4) of *Y. lipolytica* as a selection marker.

At this time, the genomic DNA of CC08-2050 was used as a template and PCR was performed using the primers of SEQ ID NO: 35 and SEQ ID NO: 36, SEQ ID NO: 37 and SEQ ID NO: 38, SEQ ID NO: 39 and SEQ ID NO: 40, SEQ ID NO: 41 and SEQ ID NO: 42, and SEQ ID NO: 43 and SEQ ID NO: 44 on the left homologous region, TEF promoter, URA3, repeat region, and right homologous region fragments, respectively. The PCR conditions consisted of denaturation at 95°C for 1 minute, annealing at 55°C for 1 minute, and polymerization at 72°C for 2 minutes, repeated for 35 cycles. The obtained DNA fragments were assembled into a single cassette through overlap extension PCR. The cassette thus constructed was introduced into the CC08-2050 strain using the heat shock method (D.-C. Chen et al., Appl Microbiol Biotechnol, 1997), and colonies formed on a solid medium (YLMM1) lacking uracil were obtained. The insertion of the cassette into the genome was confirmed using primers of SEQ **ID** NO: 45 and SEQ **ID** NO: 46. The obtained colonies were cultured on 5-FOA solid medium at 30°C for 3 days, and by obtaining colonies grown on the 5-FOA solid medium, the URA3 marker was recovered. Thus-obtained final KCCM13294P strain with increased SNQ2 protein activity was named CC08-2312.

Further, CC08-2050 in the foregoing process of constructing CC08-2312 was replaced with CC08-2507 to construct a final CC08-2507 strain with increased SNQ2 protein activity, and the final strain was named CC08-2522.

**[Table 3]**

| SEQ ID NO | Sequence (5'- 3') | PCR Product |
|---|---|---|
| 35 | ATTCTCATCAGGTAGAGCCACCCACCTTGG | Homology left arm |
| 36 | ATGCGCCGCCAACCCGGTCTCTGGTGCTTGG AGTGGTGATATGT | |
| 37 | ACATATCACCACTCCAAGCACCAGAGACCGG GTTGGCGGCGCAT | TEF promoter |
| 38 | ATGACGAGTCAGACAGGAGGCATTTGAATGAT TCTTATACTCAG | |
| 39 | CTGAGTATAAGAATCATTCAAATGCCTCCTGT CTGACTCGTCAT | URA3 |
| 40 | ATGCGCCGCCAACCCGGTCTCTTGGTGGTAT TGTGACTGGGGAT | |
| 41 | ATCCCCAGTCACAATACCACCAAGAGACCGG GTTGGCGGCGCAT | Repeat region |
| 42 | GATCGATAGGGCCGTTGTCCATTTTGAATGAT TCTTATACTCAG | |
| 43 | CTGAGTATAAGAATCATTCAAAATGGACAACG | Homology right arm |
| | GCCCTATCGATC | |
| 44 | AAATGGAAGCTCGGGTAGCCAGGGCCTCTG | |
| 45 | GATCGGGTCGGAAAGTGAGATAAA | Check forward |
| 46 | TGGGTGTTGAAGACGCCAATGTTC | Check reverse |

The aforementioned YLMM1 and 5-FOA media used had the same compositions as described in Example 1.

### Example 4. Comparative evaluation of retinol-producing/excreting ability of strains with reduced ABC3 and strains with increased ABC3

To compare the retinol-producing ability and the retinol-excreting ability of the strains (CC08-2507, CC08-2508, CC08-2312, and CC08-2522) constructed in Examples 1 to 3 with that of the control strain (CC08-2050), a flask evaluation was conducted. Each strain was inoculated to an initial OD=2 in a 250 mL corner-baffle flask containing 25 mL of YPDLU medium supplemented with 0.05% BHT (3,5-di-tert-4-butylhydroxytoluene), and cultured at 30°C and 200 rpm for 48 hours. The YPDLU medium used had the following composition:
<YPDLU>

Glucose 60 g/L, Bacto peptone 20g/L, Yeast extract 10g/L, Uracil 1g/L, Leucine 1g/L, 1M Phosphate buffer (pH 7.0) 100ml/L

To evaluate the growth level of each strain, OD value was measured at a wavelength of 600 nm using a spectrophotometer. The concentrations of retinol and retinal excreted extracellularly were quantified by mixing 0.1 mL of the supernatant in which microbial cells in the culture broth were removed after completion of culturing with 0.9 mL of acetone containing 4% BHT (Sigma), followed by analysis using HPLC equipment.

The analyzed OD values and the concentrations of retinol and retinal are as shown in Table 4 below and schematically illustrated in FIG. 1.

**[Table 4]**

| Strain | OD (A₆₀₀) | Extracellular | | n-fold | |
|---|---|---|---|---|---|
| | | Retinol (µg/L) | Retinal (µg/L) | Retinol | Retinal |
| CC08-2050 | 78 | 48,000 | 12,681 | 1.00 | 1.00 |
| CC08-2507 | 80 | 65,841 | 12,365 | 1.37 | 0.98 |
| CC08-2508 | 84 | 39,662 | 12,132 | 0.83 | 0.96 |
| CC08-2312 | 82 | 80,326 | 13602 | 1.64 | 1.07 |
| CC08-2522 | 85 | 105,227 | 20130 | 2.15 | 1.59 |

Comparing the values of CC08-2507 and CC08-2508 (experimental strains) with the values of CC08-2050 (control strain) in Table 4, retinol excreted when ABC3 protein activity was reduced (CC08-2507) increased compared to the control (CC08-2050) by 1.37-fold, while the excreted retinal was at an equivalent level. However, retinol excreted when ABC3 protein activity was increased (CC08-2508) is merely 0.83-fold of that excreted by the control (CC08-2050), while the excreted retinal was at an equivalent level.

Additionally, comparing the values of CC08-2507, CC08-2312, and CC08-2522 (experimental strains) with the values of CC08-2050 (control strain) in Table 4, retinol excreted when ABC3 protein activity was reduced (CC08-2507) and retinol excreted when SNQ2 protein activity was increased (CC08-2312) increased compared to the control (CC08-2050) by 1.37-fold and 1.64-fold, respectively, while the excreted retinal was at an equivalent level. Meanwhile, retinol excreted when ABC3 protein activity was reduced and the SNQ2 protein activity was increased simultaneously (CC08-2522) increased compared to the control (CC08-2050) by 2.15-fold. The retinol excretion was further increased compared to when ABC3 activity was reduced alone or when SNQ2 activity was increased alone. Accordingly, the simultaneous application of the reduction of ABC3 activity and the increase in SNQ2 activity may further increase the production/excretion of retinol compared to the application of each alone.

From the above results, it was confirmed that ABC3 of a *Yarrowia* sp. microorganism plays a role in retinol excretion and that reducing ABC3 activity increases retinol excretion by the microorganism. In addition, a synergistic effect on retinol excretion between the reduction of ABC3 activity and the increase of SNQ2 activity was confirmed.

As set forth above, those skilled in the art will be able to understand that the present disclosure may be embodied in other specific forms without departing from the technical spirit or essential characteristics thereof. Therefore, the embodiments described above should be construed as being exemplified and not limiting the present disclosure. It should be understood that all changes or modifications derived from the definitions and the scopes of the claims and their equivalents fall within the scope of the present disclosure.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Yarrowia lipolytica CC08-2050 | Accession number given by the
INTERNATIONAL DEPOSITARY AUTHORITY:
KCCM13294P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on December. 01. 2022 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: December. 01. 2022.

¹ Where Rule 6.4(d) applies, such date is the date on which the status of international depositary authority was
acquired.

Form BP/4 (sole page)

한국미생물보존센터 사단법인 한국종균협회
03641 서울특별시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yurim Bldg., 45, Hongjenae 2ga-gil, Seodaemun-gu, Seoul, 03641, Korea. Tel: 82-2-391-0950, 396-0950 Fax: 82-2-392-2859

## Claims

1. *A Yarrowia* sp. microorganism having retinol-producing ability in which the activity of an ABC3 (ATP-binding cassette transporter type 3) protein is reduced compared to its endogenous activity.

2. The microorganism according to claim 1, wherein the ABC3 protein comprises YALIOB02544 protein.

3. The microorganism according to claim 1, wherein the ABC3 protein comprises an amino acid sequence of SEQ **ID** NO: 1 or an amino acid sequence having 60% or more identity thereto.

4. The microorganism according to claim 1, wherein the ABC3 protein is encoded by a polynucleotide sequence of SEQ **ID** NO: 2 or a polynucleotide sequence having 60% or more identity thereto.

5. The microorganism according to claim 1, wherein the ABC3 protein is derived from *Yarrowia lipolytica.*

6. The microorganism according to claim 1, wherein the microorganism additionally exhibits increased activity of a SNQ2 protein compared to its endogenous activity.

7. The microorganism according to claim 6, wherein the SNQ2 protein comprises an amino acid sequence of SEQ ID NO: 33 or an amino acid sequence having 60% or more identity thereto.

8. The microorganism according to claim 6, wherein the SNQ2 protein is encoded by a polynucleotide sequence of SEQ ID NO: 34 or a polynucleotide sequence having 60% or more identity thereto.

9. The microorganism according to claim 1, wherein the *Yarrowia* sp. microorganism is *Yarrowia lipolytica.*

10. The microorganism according to claim 1, wherein the microorganism has increased retinol-producing ability or retinol-excreting ability compared to a non-modified *Yarrowia* sp. microorganism.

11. A method for producing retinol, comprising culturing a *Yarrowia* sp. microorganism having retinol-producing ability in which the activity of an ABC3 protein is reduced compared to its endogenous activity, in a medium.

12. The method according to claim 11, wherein the microorganism additionally exhibits increased activity of a SNQ2 protein compared to its endogenous activity.

13. The method according to claim 11, comprising recovering retinol from the cultured medium or microorganism.

14. The method according to claim 11, wherein the method, during retinol extraction, does not utilize microbial cell disruption or use dodecane as a solvent.

15. A method for preparing a *Yarrowia* sp. microorganism having retinol-producing ability, comprising reducing the activity of an ABC3 protein in a *Yarrowia* sp. microorganism having retinol-producing ability.

16. The method according to claim 15, further comprising increasing the activity of a SNQ2 protein in the microorganism.

17. A method for increasing retinol excretion, comprising reducing the activity of an ABC3 protein in a *Yarrowia* sp. microorganism having retinol-producing ability.

18. The method according to claim 17, further comprising increasing the activity of a SNQ2 protein in the microorganism.

19. A composition for producing retinol, comprising one or more of a *Yarrowia* sp. microorganism having retinol-producing ability in which the activity of an ABC3 protein is reduced compared to its endogenous activity, and a culture thereof.

20. The composition according to claim 19, wherein the microorganism additionally exhibits increased activity of a SNQ2 protein compared to its endogenous activity.

21. Use of a *Yarrowia* sp. microorganism in which the activity of an ABC3 protein is reduced compared to its endogenous activity, or a culture thereof for retinol production.

22. The use according to claim 21, wherein the microorganism additionally exhibits increased activity of a SNQ2 protein compared to its endogenous activity.
